**Europäisches Patentamt**

**European Patent Office**

**Office européen des brevets**

(19)

(11) Veröffentlichungsnummer: **0 252 908 B1**

(12) # EUROPÄISCHE PATENTSCHRIFT

(45) Veröffentlichungstag der Patentschrift:
16.08.89

(21) Anmeldenummer: 86900754.2

(22) Anmeldetag: 03.01.86

(86) Internationale Anmeldenummer:
PCT/EP 86/00001

(87) Internationale Veröffentlichungsnummer:
WO 86/04803 (28.08.86 Gazette 86/19)

(51) Int. Cl.⁴: **A 61 B 7/02**

(54) DOPPELSCHLAUCHSTETHOSKOP.

(30) Priorität: 21.02.85 DE 8504877 U

(43) Veröffentlichungstag der Anmeldung
20.01.88 Patentblatt 88/3

(45) Bekanntmachung des Hinweises auf die Patenterteilung
16.08.89 Patentblatt 89/33

(84) Benannte Vertragsstaaten:
AT CH DE FR GB LI

(56) Entgegenhaltungen:
AU-B- 504 509
DE-A- 2 631 154
DE-B- 1 267 378
US-A- 3 193 047
US-A- 3 712 409

(73) Patentinhaber: WILHELM HASELMEIER GMBH & CO.,
Vaihinger Strasse 48, D-7000 Stuttgart 80 (DE)

(72) Erfinder: GABRIEL, Jochen, Im Falkenrain 1,
D-7000 Stuttgart 1 (DE)

(74) Vertreter: Raible, Hans, Dipl.-Ing., Lenbachstrasse 32,
D-7000 Stuttgart 1 (DE)

ACTORUM AG

Beschreibung

Die Erfindung betrifft ein Doppelschlauchstethoskop mit einem Bruststück, das einen Hörkopf grösseren Durchmessers, z. B. zum Auskultieren der tiefen Herztöne, einen Einkammer-Hörkopf kleineren Durchmessers, z. B. zum Auskultieren der hohen Herztöne, und eine Umschaltvorrichtung aufweist, mittels deren entweder der eine oder der andere Hörkopf mit dem Doppelschlauch verbindbar ist.

Derartige Stethoskope sind bekannt aus den US-A 1 671 936, US-A 3 035 656, US-A 3 193 047, US-A 3 712 409 und AU-B 504 509. Dies sind Stethoskope mit einem Bruststück, das einen Hörkopf grösseren Durchmessers, z. B. zum Auskultieren der tieferen Herztöne, einen Einkammer-Hörkopf kleineren Durchmessers, z. B. zum Auskultieren der hohen Herztöne, und eine Umschaltvorrichtung aufweist, mittels deren entweder der Hörkopf grösseren Durchmessers oder der Hörkopf kleineren Durchmessers mit einem Einfachschlauch oder einem Doppelschlauch verbunden werden kann. Der Einfach- oder Doppelschlauch führt dann zu dem Ohrbügel oder den Ohroliven. Diese Stethoskope ermöglichen keine stereophonische Auskultation durch den Arzt.

Aus der DE-A 2 631 154 kennt man ein Stethoskop mit einem einzigen Hörkopf. In diesem sind zwei in ihrer Grundform paraboloidartige Schalltrichter vorgesehen, d.h. dieser Hörkopf stellt einen Doppelkammer-Hörkopf dar. Von jeder Kammer wird der Schall durch einen Schlauch zum zugeordneten Ohr geführt. Das ermöglicht eine stereophonische Auskultation. Will der Arzt jedoch speziell die hohen Töne auskultieren, so muss er noch ein zweites Stethoskop mit einem Hörkopf kleineren Durchmessers mit sich führen.

Aus der DE-B 1 267 378 ist ein Stethoskop zur stereophonischen Auskultation bekannt, welches einen Hörkopf aufweist, der durch Druck auf die Membran selektiv von einem Einkammer-Hörkopf in einen Doppelkammer-Hörkopf umschaltbar ist. Jede Kammer hat ihren eigenen Schlauchanschluss. Auch hier muss der Arzt zum Auskultieren der hohen Töne ein zweites Stethoskop mit einem Hörkopf kleineren Durchmessers mit sich führen.

Ferner kennt man aus der US-A 3 938 615 ein Doppelschlauchstethoskop, das einen Hörkopf grösseren Durchmessers, einen Einkammer-Hörkopf kleineren Durchmessers und eine Umschaltvorrichtung aufweist. Dieses Stethoskop hat einen Doppelschlauch zur stereophonischen Auskultation, jedoch ist der Hörkopf grösseren Durchmessers nicht als Doppelkammer-Hörkopf ausgebildet. Ferner wird durch die Umschaltvorrichtung in der Schaltstellung für den Betrieb des Hörkopfs kleineren Durchmessers eine Verbindung mit dem Hörkopf grösseren Durchmessers hergestellt, weshalb von diesem Störgeräusche aufgenommen und an das Ohr des Arztes weitergeleitet werden. Dies behindert die Auskultation.

Deshalb ist es eine Aufgabe der Erfindung, die Stethoskope mit zwei Hörköpfen zu verbessern.

Nach der Erfindung wird diese Aufgabe bei einem eingangs genannten Stethoskop dadurch gelöst, dass der Hörkopf grösseren Durchmessers als Doppelkammer-Hörkopf mit zwei Hörkammern ausgebildet ist, welch letztere durch die Umschaltvorrichtung jeweils mit dem zugeordneten Schlauch des Doppelschlauchs verbindbar sind, und dass die Hörkammer des Einkammer-Hörkopfs durch die Umschaltvorrichtung mit beiden Schläuchen des Doppelschlauchs verbindbar ist, wobei in der Schaltstellung, in der der Doppelkammer-Hörkopf aktiviert ist, dessen Hörkammern nicht mit der Hörkammer des Einkammer-Hörkopfs in Verbindung stehen, und wobei in der Schaltstellung, in der der Einkammer-Hörkopf aktiviert ist, dessen Hörkammer nicht mit den Hörkammern des Doppelkammer-Hörkopfs in Verbindung steht. Man erhält so in der Schaltstellung der Umschaltvorrichtung für den Einkammer-Hörkopf die Schallfortleitung durch beide Schläuche, ohne dass dabei eine Verbindung mit dem Doppelkammer-Hörkopf entsteht. In der Schaltstellung der Umschaltvorrichtung für den Doppelkammer-Hörkopf wird der Schall aus der einen Kammer durch den einen Schlauch und der Schall aus der anderen Kammer durch den anderen Schlauch fortgeleitet, so dass die Lokalisierung der Schallquelle für den Arzt verbessert wird und Zusammensetzung und Qualität des vom Arzt wahrgenommenen Schalles besser werden. In der Schaltstellung für den Doppelkammer-Hörkopf ist ebenfalls keine Verbindung zum Einkammer-Hörkopf gegeben.

Mit besonderem Vorteil geht man nach der Erfindung so vor, dass die Umschaltvorrichtung ein drehbares Umschaltglied aufweist, welches am Bruststück zwischen den beiden Hörköpfen angeordnet und durch Drehung relativ zu diesen betätigbar ist. Ein solches drehbares Umschaltglied ist einfach zu bedienen und ermöglicht kurze Schallfortleitungskanäle so dass in ihm sehr geringe Signalverluste entstehen. Auch ist seine Bedienung sehr sinnfällig, und diese kann z. B. durch einfache Markierungen, welche die jeweilige Schaltstellung anzeigen, zusätzlich benutzerfreundlich gemacht werden. Ein einfacher Aufbau eines solchen Stethoskops ergibt sich dadurch, dass die beiden Hörköpfe durch ein Verbindungsglied im Abstand voneinander gehalten und starr miteinander verbunden sind, und dass das Umschaltglied um dieses Verbindungsglied herum angeordnet und relativ zu ihm verdrehbar ist.

Dabei wird das Stethoskop nach der Erfindung mit besonderem Vorteil so ausgebildet, dass das drehbare Umschaltglied mit mindestens einem Hörköpf oder mit dem Verbindungsglied in Flächenkontakt steht, und dass die miteinander in Kontakt stehenden Oberflächen nach Art eines Schieberventils als Steuerflächen zur Umschaltung mindestens eines zur Weiterleitung von Schall dienenden Kanals ausgebildet sind.

Besonders einfach ist dabei ein Aufbau des Stethoskops dergestalt, dass der Doppelschlauch mit dem Umschaltglied verbunden ist.

Eine sehr einfache Ausgestaltung der Erfindung ergibt sich ferner dadurch, dass am Umschaltglied mindestens eine Steuerfläche vorgesehen ist, welche zwei Öffnungen aufweist, von denen jede mit einem zugeordneten Schlauch des Doppelschlauchs verbunden ist.

Eine besonders vorteilhafte Weiterbildung der Erfindung zeichnet sich dadurch aus, dass der Doppelkammer-Hörkopf eine zu einer ersten Steuerfläche des Umschaltglieds komplementäre Steuerfläche aufweist, in der zwei jeweils zu einer der beiden Hörkammern führende Verbindungen münden, und dass in mindestens einer der Drehstellungen des Umschaltglieds diese Verbindungen des Doppelkammer-Hörkopfs jeweils mit einer zugeordneten Öffnung des Umschaltgliedes in Verbindung bringbar sind.

Für den Einkammer-Hörkopf ergibt sich eine bevorzugte Lösung in der Weise, dass das Verbindungsglied, vorzugsweise an seinem Aussenumfang, eine Steuerfläche mit zwei Öffnungen aufweist, welche beide mit der Hörkammer des Einkammer-Hörkopfs verbunden sind, und dass das Umschaltglied eine zu dieser Steuerfläche komplementäre zweite Steuerfläche mit zwei jeweils mit einem Schlauch des Doppelschlauchs verbundenen Steueröffnungen aufweist, die in mindestens einer Drehstellung des Umschaltglieds mit den genannten Öffnungen in Verbindung bringbar sind.

Dabei wird das Stethoskop bevorzugt so ausgebildet, dass in der mindestens einen Drehstellung, in der die beiden Hörkammern des Doppelkammer-Hörkopfs durch das Umschaltglied jeweils mit dem zugeordneten Schlauch des Doppelschlauchs verbunden sind, die Öffnungen der am Verbindungsglied vorgesehenen Steuerfläche durch die zweite Steuerfläche des Umschaltglieds blockiert sind.

Ebenso wird das Stethoskop bevorzugt so ausgebildet, dass in der mindestens einen Drehstellung des Umschaltglieds, in der die Hörkammer des Einkammer-Hörkopfs mit den beiden Schläuchen des Doppelschlauchs verbunden ist, die zu den beiden Hörkammern führenden Verbindungen des Doppelkammer-Hörkopfs durch die erste Steuerfläche des Umschaltgliedes blockiert sind.

Ebenfalls zur Abschirmung störender Schallquellen geht man in bevorzugter Weise so vor, dass zwischen mindestens einem Hörkopf und dem Umschaltglied ein ausserhalb der Steueröffnungen angeordneter Dichtring, vorzugsweise aus einem gleitfähigen Material und insbesondere aus Polytetrafluorethylen, angeordnet ist.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel, sowie aus den Unteransprüchen 12 und 13. Es zeigt (alles in vergrössertem Massstab):

Fig. 1 eine Seitenansicht eines Bruststücks für ein erfindungsgemässes Stethoskop,

Fig. 2 eine Draufsicht von oben auf das Bruststück der Fig. 1, wobei der Doppelschlauch angedeutet ist,

Fig. 3 einen Schnitt durch das Bruststück, gesehen längs der Linie III-III der Fig. 2, wobei das Umschaltglied teilweise nicht geschnitten dargestellt ist,

Fig. 4 eine Draufsicht auf das Bruststück, gesehen längs des Pfeiles A der Fig. 3,

Fig. 5 eine Darstellung analog Fig. 3, welche eine andere Schaltstellung des Umschaltgliedes zeigt,

Fig. 6 einen Schnitt, gesehen längs der Linie VI-VI der Fig. 5,

Fig. 7 eine Seitenansicht des Umschaltgliedes entsprechend der Darstellung in den Fig. 3 und 5, und gesehen in Richtung des Pfeiles B der Fig 8,

Fig. 8 einen Schnitt, gesehen längs der Linie VIII-VIII der Fig. 9,

Fig. 9 eine Draufsicht auf das Umschaltglied, gesehen längs des Pfeiles C der Fig. 8, und

Fig. 10 einen Schnitt durch das Umschaltglied, gesehen etwa längs seiner Mittelebene.

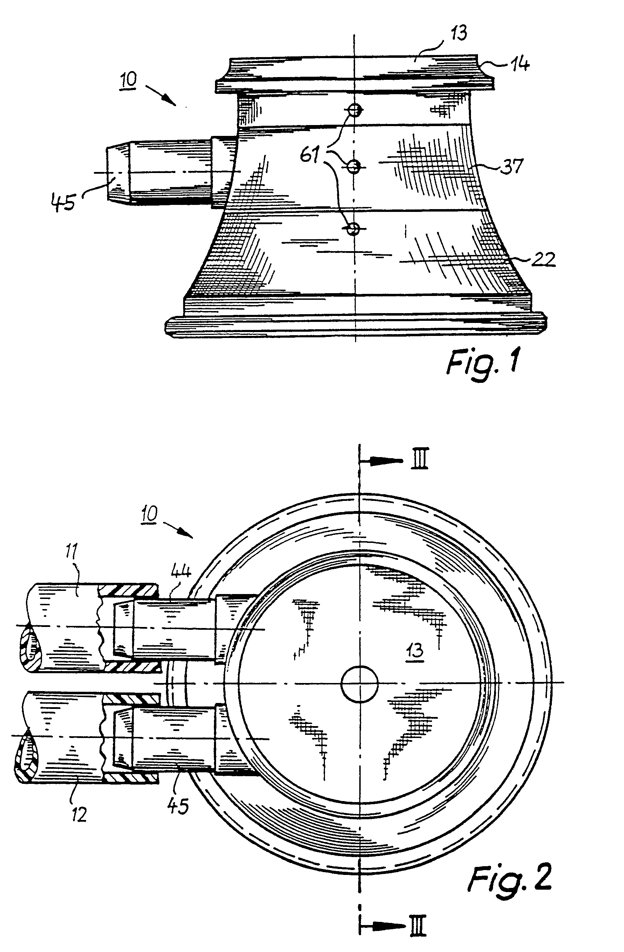

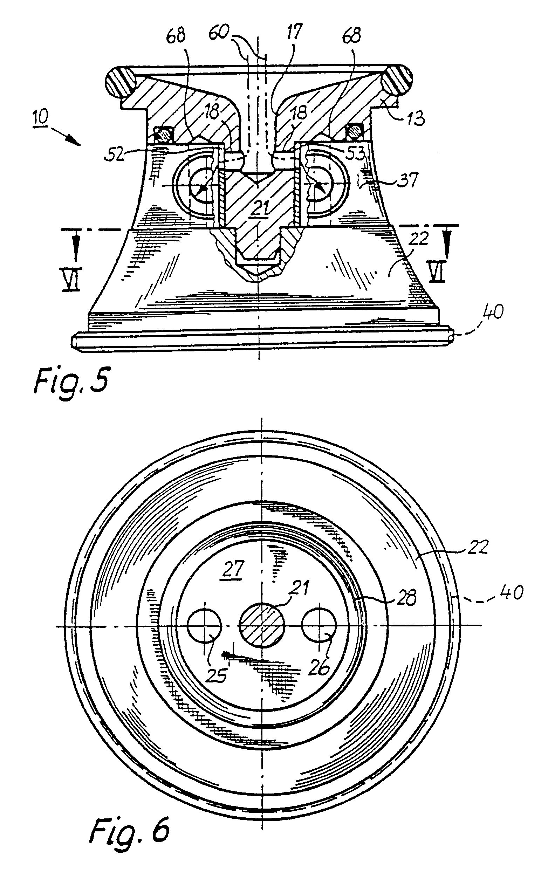

Die Fig. 1-3 und 5 zeigen ein Bruststück 10 eines Stethoskops, dessen beide Anschlussschläuche 11 und 12 (Fig. 2) in Form eines (nicht dargestellten) Doppelschlauchs zu einem (nicht dargestellten) Ohrbügel bekannter Form geführt sind, um diesem den vom Bruststück 10 aufgenommenen Schall zuzuführen. Das Bruststück hat einen Hörkopf 13 kleineren Durchmessers, auf dessen Aussenumfang in einer Ringnut 14 ein O-Ring 15 befestigt ist, um diesen Hörkopf schalldicht gegen den Körper eines Patienten, z. B. eines Kindes, pressen zu können. Die Hörkammer bzw. der Schalltrichter 16 dieses Hörkopfs 13 geht über in einen zentralen Kanal 17, von dem, wie in Fig. 5 dargestellt, zwei Seitenkanäle 18 abzweigen, deren äussere Enden als Steueröffnungen fungieren und im als Steuerfläche dienenden Aussenumfang eines zylindrischen Verbindungsgliedes 21 münden, das den Hörkopf 13 in einer vorgegebenen Winkellage mit dem Hörkopf grösseren Durchmessers 22 starr verbindet, z. B. wie dargestellt durch eine Pressverbindung 19.

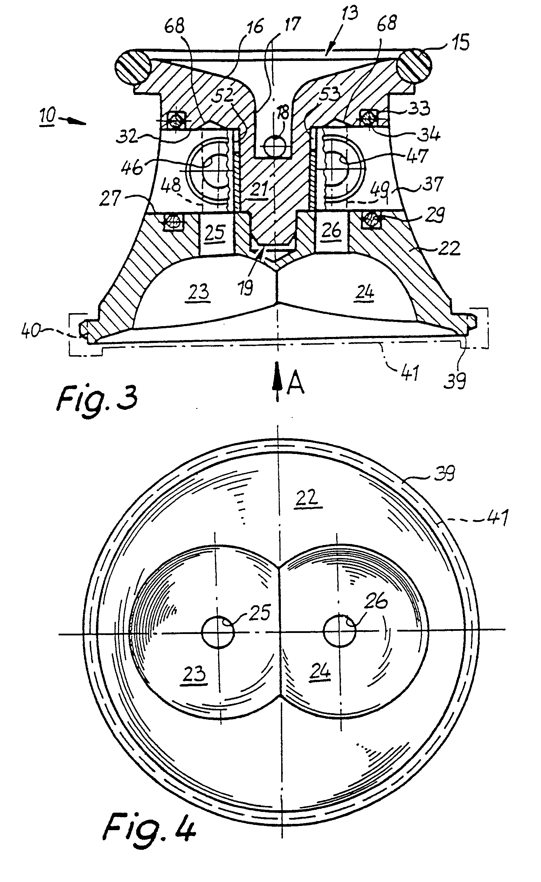

Wie die Fig. 3 und 4 zeigen, hat der Hörkopf 22 zwei im Abstand voneinander angeordnete, etwa kugelige oder paraboloidförmige Hörkammern 23 und 24, deren Mittenabstand grösser ist als 10 mm. Ihre Form und Anordnung ergibt sich in allen Einzelheiten aus den Fig. 3 und 4 und braucht, da an sich bekannt, nicht näher erläutert zu werden. Von der Kammer 23 geht eine Verbindung 25 und von der Kammer 24 eine Verbindung 26 zu einer ebenen Steuerfläche 27 auf der Rückseite des Hörkopfs 22, welche in Fig. 6 in der Draufsicht dargestellt ist. In dieser Steuerfläche 27 befindet sich auch – ausserhalb der Verbindungen 25 und 26 – eine Ringnut 28 zur Aufnahme eines O-Rings 29 aus PTFE (Polytetrafluorethylen). Ebenso befindet sich, etwa spiegelbildlich gegenüberliegend, in einer Fläche 32 auf der Rückseite des Hörkopfs 13 eine Ringnut 33 zur Aufnahme eines O-Rings 34 aus PTFE. Selbstverständlich können die Nuten 28 und 33 auch andere, von der Kreisform abweichende Formen haben. Die O-Ringe 29 und 34 haben vor allem die Aufgabe, ein geschmeidiges Gleiten eines ringförmigen Umschaltgliedes 37 zwischen den beiden Flächen 27 und 32 zu ermöglichen. Ausserdem verhindern sie das unerwünschte Eindringen von Fremdschall in die Hörköpfe, und sie verhindern umgekehrt, dass Schall aus den Hörköpfen verlorengeht. — Der Hörkopf 22 hat an seinem Aussenumfang ein Aussengewinde 40, auf das in üblicher Weise ein Träger mit einer Membran 41 aufgeschraubt ist. Ferner hat er dort eine ringförmige, ebene Anlagefläche 39 für die Membran 41, um ein Verspannen der Membran 41 zu verhindern.

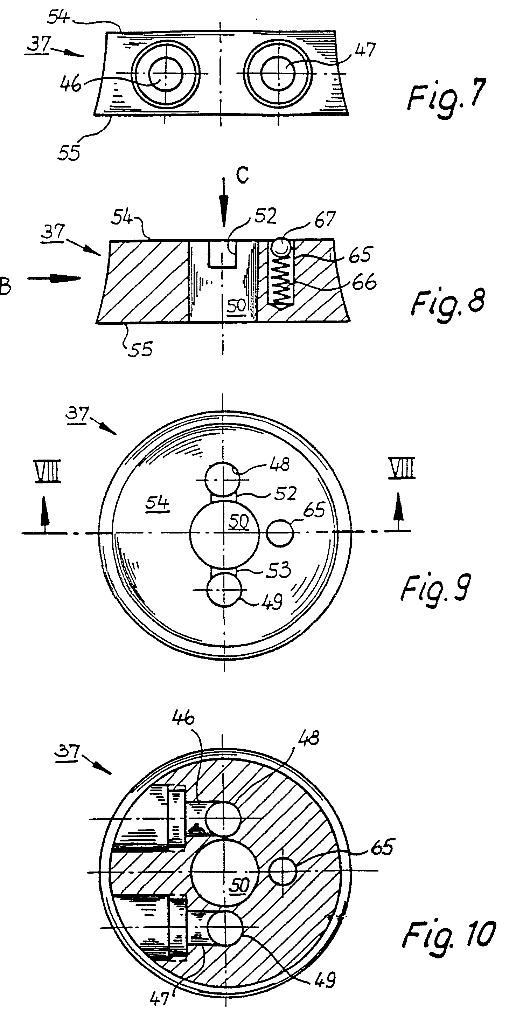

Das Umschaltglied 37 ist in den Fig. 7-10 näher dargestellt. Es hat seitlich zwei eingeschraubte Anschlussbuchsen 44 und 45 (Fig. 2) für den Doppelschlauch 11 und 12, die sich in Kanälen 46 bzw. 47 (Fig. 7 und 10) fortsetzen. Diese münden, etwa in der Mitte des Umschaltglieds 37, in zwei axiale Kanäle 48 bzw. 49, die parallel zu einer zylindrischen Mittelausnehmung 50 des Umschaltglieds 37 verlaufen und das Umschaltglied 37 in seiner ganzen Länge durchdringen. Der Innendurchmesser der Mittelausnehmung 50 entspricht etwa dem Aussendurchmesser des Verbindungsglieds 21, so dass das Umschaltglied 37 auf dem Verbindungsglied 21 drehbar geführt ist, wobei die aufeinander gleitenden Flächen als Steuerflächen dienen.

Wie die Fig. 8 und 9 zeigen, sind die Kanäle 48 und 49 durch Steuerschlitze 52 bzw. 53 mit der Mittelausnehmung 50 verbunden. Diese Schlitze erstrecken sich jeweils über einen Winkel von etwa 30°, und sie erstrecken sich von der der Fläche 32 zugewandten ebenen Stirnseite 54 (die andere ebene Stirnseite ist mit 55 bezeichnet) des Umschaltglieds 37 über einen Teil der axialen Erstreckung der Mittelausnehmung 50, z. B. wie dargestellt über etwa ein Drittel.

Befindet sich das Umschaltglied 37 relativ zu den Hörköpfen 13 und 22 in der Stellung gemäss Fig. 3, so sind die Steueröffnungen 18 durch die Innenseite der Mittelausnehmung 50 verschlossen, da die Steuerschlitze 52 und 53 relativ zu diesen Offnungen 18 um 90° verdreht sind. Es besteht also keine Verbindung vom Hörkopf 13 zu den Schläuchen 11 und 12.

Dagegen sind aber in Fig. 3 die Kammern 23 und 24 des Hörkopfes 22 jeweils mit einem zugeordneten Schlauch 11 bzw. 12 verbunden, wie folgt: Die Kammer 23 ist über die Verbindung 25 mit dem axialen Kanal 48 und über diesen mit dem Kanal 46 und dem Schlauch 11 verbunden. Ebenso ist die Kammer 24 über die Verbindung 26, den axialen Kanal 49 und den Kanal 47 mit dem Schlauch 12 verbunden. Es wird also für jedes Ohr ein Signal übertragen, d. h. jedes Ohr hört nur das Signal von der ihm zugeordneten Hörkammer des Doppelkammer-Hörkopfs 22.

Umgekehrt sind bei der Schaltstellung nach Fig. 5 die Verbindungen 25 und 26 des Hörkopfs 22 durch die gegenüberliegende Steuerfläche 55 des Umschaltglieds 37 versperrt. Hingegen stehen in dieser Drehstellung die Steueröffnungen 18 in Verbindung mit den Steuerschlitzen 52 bzw. 53, und über diese mit den axialen Kanälen 48 bzw. 49 und den Schläuchen 11 bzw. 12. Der Schall vom Hörkopf 13 kann also, wie durch die strichpunktierten Linien 60 der Fig. 5 angedeutet, von diesem Hörkopf in beide Schläuche 11 und 12 des Doppelschlauches gelangen und von dort zu beiden Ohren gelangen, d. h. beide Ohren hören hier dasselbe.

Auf diese Weise erhält man also je nach Drehstellung des Umschaltgliedes 37 entweder eine Verbindung vom Hörkopf 13 zu beiden Schläuchen 11 und 12, oder vom Hörkopf 22 in der Weise, dass jede Kammer 23, 24 desselben mit nur einem Schlauch des Doppelschlauches 11, 12 verbunden wird.

Gemäss Fig. 1 können am Bruststück 10 Markierungen 61 vorgesehen werden, um die jeweilige Schaltstellung anzuzeigen. In der Praxis dreht der Arzt einfach das Umschaltglied 37, bis er den Schall hört. Hierzu sind 4 Raststellungen für das Umschaltglied 37 vorgesehen, und dieses hat hierzu eine Ausnehmung 65 in Form einer Sackbohrung, in der eine von einer Feder 66 beaufschlagte Kugel 67 angeordnet ist. In der Fläche 32 des monauralen Hörkopfs 13 befinden sich vier um je 90° versetzte Rastvertiefungen 68, von denen in den Fig. 3 und 5 jeweils zwei dargestellt sind, und in diese rastet jeweils die Kugel 67 ein, wenn sich das Umschaltglied 37 in einer seiner Schaltstellungen befindet.

Selbstverständlich sind im Rahmen der Erfindung vielfältige Modifikationen möglich, ohne den Grundgedanken der Erfindung zu verlassen.

Patentansprüche

1. Doppelschlauchstethoskop mit einem Bruststück (10), das einen Hörkopf grösseren Durchmessers (22), z. B. zum Auskultieren der tiefen Herztöne, einen Einkammer-Hörkopf kleineren Durchmessers (13), z. B. zum Auskultieren der hohen Herztöne, und eine Umschaltvorrichtung (37) aufweist, mittels deren entweder der eine oder der andere Hörkopf mit dem Doppelschlauch (11, 12) verbindbar ist, dadurch gekennzeichnet, dass der Hörkopf grösseren Durchmessers (22) als Doppelkammer-Hörkopf mit zwei Hörkammern (23, 24) ausgebildet ist, welch letztere durch die Umschaltvorrichtung (37) jeweils mit dem zugeordneten Schlauch (11 oder 12) des Doppelschlauchs verbindbar sind, und dass die Hörkammer (16) des Einkammer-Hörkopfs (13) durch die Umschaltvorrichtung (37) mit beiden Schläuchen (11 und 12) des Doppelschlauchs verbindbar ist, wobei in der Schaltstellung, in der der Doppelkammer-Hörkopf (22) aktiviert ist, dessen Hörkammern (23, 24) nicht mit der Hörkammer (16) des Einkammer-Hörkopfs (13) in Verbindung stehen, und wobei in der Schaltstellung, in der der Einkammer-Hörkopf (13) aktiviert ist, dessen Hörkammer (16) nicht mit den Hörkammern (23, 24) des Doppelkammer-Hörkopfs (22) in Verbindung steht.

2. Stethoskop nach Anspruch 1, dadurch gekennzeichnet, dass die Umschaltvorrichtung ein drehbares Umschaltglied (37) aufweist, welches am Bruststück (10) zwischen den beiden Hörköpfen (13, 22) angeordnet und durch Drehung relativ zu diesen betätigbar ist.

3. Stethoskop nach Anspruch 2, dadurch gekennzeichnet, dass die beiden Hörköpfe (13, 22) durch ein Verbindungsglied (21) im Abstand voneinander gehalten und starr miteinander verbunden sind, und dass das Umschaltglied (37) um dieses Verbindungsglied (21) angeordnet und relativ zu ihm verdrehbar ist.

4. Stethoskop nach Anspruch 3, dadurch gekennzeichnet, dass das drehbare Umschaltglied (37) mit mindestens einem Hörkopf oder mit dem Verbindungsglied (21) in Flächenkontakt steht, und dass die miteinander in Kontakt stehenden Oberflächen nach Art eines Schieberventils als Steuerflächen (27, 50, 55) zur Umschaltung mindestens eines zur Weiterleitung von Schall dienenden Kanals (25, 48, 26, 49, 18, 52, 53) ausgebildet sind.

5. Stethoskop nach mindestens einem der Ansprüche 2 bis 4, dadurch gekennzeichnet, dass der Doppelschlauch (11, 12) mit dem Umschaltglied (37) verbunden ist.

6. Stethoskop nach Anspruch 4 und 5, dadurch gekennzeichnet, dass am Umschaltglied (37) eine Steuerfläche (55) vorgesehen ist, welche zwei Offnungen (48, 49) aufweist, von denen jede mit einem zugeordneten Schlauch (11 bzw. 12) des Doppelschlauchs verbunden ist.

7. Stethoskop nach Anspruch 6, dadurch gekennzeichnet, dass der Doppelkammer-Hörkopf (22) eine zu einer ersten Steuerfläche (55) des Umschaltglieds (37) komplementäre Steuerfläche (27) aufweist, in der zwei jeweils zu einer der beiden Horkammern (23, 24) führende Verbindungen (25, 26) munden, und dass in mindestens einer der Drehstellungen (Fig. 3) des Umschaltgliedes (37) diese Verbindungen (25, 26) des Doppelkammer-Hörkopfs (22) jeweils mit einer zugeordneten Offnung (48 bzw. 49) des Umschaltgliedes (37) in Verbindung bringbar sind.

8. Stethoskop nach Anspruch 6 oder 7, dadurch gekennzeichnet, dass das Verbindungsglied (21), vorzugsweise an seinem Aussenumfang, eine Steuerflache mit zwei Offnungen (18) aufweist, welche beide mit der Hörkammer (16) des Einkammer-Horkopfs (13) verbunden sind, und dass das Umschaltglied (37) eine zu dieser Steuerfläche komplementäre zweite Steuerfläche (50) mit zwei jeweils mit einem Schlauch (11 bzw. 12) des Doppelschlauchs verbundenen Steueröffnungen (52, 53) aufweist, die in mindestens einer Drehstellung (Fig. 5) des Umschaltglieds (37) mit den genannten Öffnungen (18) in Verbindung bringbar sind.

9. Stethoskop nach den Ansprüchen 7 und 8, dadurch gekennzeichnet, dass in der mindestens einen Drehstellung (Fig. 3), in der die beiden Hörkammern (23, 24) des Doppelkammer-Hörkopfs (22) durch das Umschaltglied (37) jeweils mit dem zugeordneten Schlauch (11 bzw. 12) des Doppelschlauchs verbunden sind, die Öffnungen (18) der am Verbindungsglied (21) vorgesehenen Steuerfläche durch die zweite Steuerfläche (50) des Umschaltglieds (37) blockiert sind.

10. Stethoskop nach den Ansprüchen 7 und 8, dadurch gekennzeichnet, dass in der mindestens einen Drehstellung (Fig. 5) des Umschaltglieds (37), in der die Hörkammer (16) des Einkammer-Hörkopfs (13) mit den beiden Schläuchen (11, 12) des Doppelschlauchs verbunden ist, die zu den beiden Hörkammern (23, 24) führenden Verbindungen (25, 26) des Doppelkammer-Hörkopfs (22) durch die erste Steuerfläche (55) des Umschaltglieds (37) blockiert sind.

11. Stethoskop nach mindestens einem der Ansprüche 1-10, dadurch gekennzeichnet, dass zwischen mindestens einem Hörkopf (13, 22) und dem Umschaltglied (37) ein ausserhalb der Steueröffnungen (18, 48, 49) angeordneter Dichtring (29, 34), vorzugsweise aus einem gleitfähigen Material und insbesondere aus Polytetrafluorethylen, angeordnet ist.

12. Stethoskop nach mindestens einem der Ansprüche 2-11, dadurch gekennzeichnet, dass eine Rastvorrichtung (65-68) vorgesehen ist, welche das Umschaltglied (37) in bestimmten Drehstellungen einrasten lässt, wobei vorzugsweise das Umschaltglied (37) und die Hörköpfe (13, 22) an ihrem Aussenumfang mit Markierungen (61), für die verschiedenen Drehstellungen versehen sind (Fig. 1).

13. Stethoskop nach mindestens einem der vorhergehenden Ansprüche, dadurch gekennzeichnet, dass die Horkammern (23, 24) des Doppelkammer-Hörkopfs (22) jeweils als Ausnehmungen etwa in Form einer Kugel oder eines Paraboloids ausgebildet sind, welche Ausnehmungen vorzugsweise einen Mittenabstand von mehr als 10 mm aufweisen und welche sich bevorzugt schneiden

## Claims

1. Double-tube stethoscope having a chestpiece (10) which has an auscultation head of relatively large diameter (22), for example for auscultation of the low heart sounds, a single chamber auscultation head of relatively small diameter (13), for example for auscultation of the high heart sounds, and a switchover device (37) by means of which either one or the other auscultation head can be connected to the double tube (11, 12), characterized in that the auscultation head of relatively large diameter (22) is designed as a double-chamber auscultation head having two auscultation chambers (23, 24) which latter are each connectable by the switchover device (37) to the associated tube (11 or 12) of the double-tube, and in that the auscultation chamber (16) of the single-chamber auscultation head (13) is connectable by the switchover device (37) to both tubes (11 and 12) of the double tube, where the auscultation chambers (23, 24) of the double-chamber auscultation head (22), in the setting in which it is activated, are not connected to the auscultation chamber (16) of the single chamber auscultation head (13), and where the auscultation chamber (16) of the single-chamber auscultation head (13), in the setting in which it is activated, is not connected to the auscultation chambers (23, 34) of the double-chamber auscultation head (22).

2. Stethoscope according to Claim 1, characterized in that the switchover device comprises a rotatable switchover member (37) which is located on the chestpiece (10) between two auscultation heads (13, 22) and can be actuated by rotation relative to the latter.

3. Stethoscope according to Claim 2, characterized in that the two auscultation heads (13, 22) are connected rigidly together by a connecting member (21) holding them at a distance apart, and in that the switchover member (37) is located around this connecting member (21) and is rotatable relative to it.

4. Stethoscope according to Claim 3, characterized in that the rotatable switchover member (37) is in surface contact with at least one auscultation head or with the connecting member (21), and in that the surfaces which are in contact with one another are designed in the manner of a sliding valve as control surfaces (27, 50, 55) for switching over at least one channel (25, 48, 26, 49, 18, 52, 53) serving to conduct sound

5. Stethoscope according to at least one of Claims 2 to 4, characterized in that the double-tube (11, 12) is connected to the switchover member (37).

6. Stethoscope according to Claims 4 and 5, characterized in that a control surface (55) is provided on the switchover member (37) and has two openings (48, 49) each of which is connected to an associated tube (11 or 12) of the double-tube.

7. Stethoscope according to Claim 6, characterized in that the double-chamber auscultation head (22) has a control surface (27) which is complementary to a first control surface (55) of the switchover member (37) and into which two connections (25, 26) open, each of which leads to one of the two auscultation chambers (23, 24), and in that each of these connections (25, 26) of the double-chamber auscultation head (22) is adapted to be connected to an associated opening (48 or 49) of the switchover member (37) in at least one of the rotational positions (Fig. 3) of the switchover member (37).

8. Stethoscope according to Claim 6 or 7, characterized in that the connecting member (21) comprises, preferably on its periphery, a control surface having two openings (18) both of which are connected to the auscultation chamber (16) of the single chamber auscultation head (13) and in that the switchover member (37) comprises a second control surface (50) which is complementary to this control surface and has two control openings (52, 53) each of which is connected to one tube (11 or 12) of the double-tube and which is adapted to be connected to the said openings (18) in at least one rotational position (Fig. 5) of the switchover member (37).

9. Stethoscope according to Claims 7 and 8, characterized in that, in the at least one rotational position (Fig. 3) in which the two auscultation chambers (23, 24) of the double-chamber auscultation head (22) are each connected by the switchover member (37) to the associated tube (11 or 12) of the double-tube, the openings (18) in the control surface provided on the connecting member (21) are blocked by the second control surface (50) of the switchover member (37).

10. Stethoscope according to Claims 7 and 8, characterized in that, in the at least one rotational position (Fig. 5) of the switchover member (37) in which the auscultation chamber (16) of the single-chamber auscultation head (13) is connected to the two tubes (11, 12) of the double-tube, the connections (25, 26) of the double chamber auscultation head (22) which lead to the two auscultation chambers (23, 24) are blocked by the first control surface (55) of the switchover member (37).

11. Stethoscope according to at least one of Claims 1-10, characterized in that there is located between at least one auscultation head (13, 22) and the switchover member (37) a sealing ring (29, 34) which is located outside the control openings (18, 48, 49) and is preferably composed of a low-friction material and, in particular, of polytetrafluoroethylene.

12. Stethoscope according to at least one of Claims 2-11, characterized in that there is provided a detent device (65-68) which allows the switchover member (37) to lock in certain rotational positions, there preferably being provided on the periphery of the switchover member (37) and the auscultation heads (13, 22) marks (61) for the various rotational positions (Fig. 1).

13. Stethoscope according to at least one of the preceding claims, characterized in that the auscultation chambers (23, 24) of the double-chamber auscultation head (22) are designed as recesses approximately in the form of a sphere or of a paraboloid, which recesses are preferably more than 10 mm apart centre-to-centre and which preferably intersect.

## Revendications

1. Stéthoscope à tube double souple, comprenant une pièce d'auscultation (10), qui comporte une tête d'écoute de plus grand diamètre (22), servant par exemple à l'auscultation des bruits de basse fréquence du cœur, une tête d'écoute à une chambre (13) de plus petit diamètre, servant par exemple à l'auscultation des bruits aigus du cœur, et un dispositif de commutation (37) au moyen duquel l'une ou l'autre des têtes d'écoute peut être reliée au tube double (11, 12), caractérisé en ce que la tête d'écoute (22) de plus grand diamètre est constituée par une tête d'écoute à chambre double comprenant deux chambres d'écoute (23, 24), lesquelles peuvent être chacune reliées par le dispositif de commutation (37) au tuyau correspondant (11 ou 12) du tube double, et en ce que la chambre d'écoute (16) de la tête d'écoute (13) à une chambre peut être reliée par le dispositif de commutation (37) aux deux tuyaux (11 et 12) du tube double, et, dans la position de commutation dans laquelle la tête d'écoute (22) à chambre double est en service, ses chambres d'écoute (23, 24) ne sont pas reliées à la chambre d'écoute (16) de la tête d'écoute (13) à une chambre, alors que, dans la position de commutation dans laquelle la tête d'écoute (13) à une chambre est en service, sa chambre d'écoute (16) n'est pas reliée aux chambres d'écoute (23, 24) de la tête d'écoute (22) à chambre double.

2. Stéthoscope selon la revendication 1, caractérisé en ce que le dispositif de commutation comporte un élément de commutation rotatif (37), qui est monté sur la pièce d'auscultation (10) entre les deux têtes d'écoute (13, 22) et qui est actionnable par rotation par rapport à elles.

3. Stéthoscope selon la revendication 2, caractérisé en ce que les deux têtes d'écoute (13, 22) sont maintenues écartées l'une de l'autre et rigidement reliées entre elles par un élément de jonction (21), et en ce que l'élément de commutation (37) est disposé autour de cet élément de jonction et est capable de tourner par rapport à lui.

4. Stéthoscope selon la revendication 3, caractérisé en ce que l'élément de commutation rotatif (37) est en contact superficiel avec au moins une tête d'écoute ou avec l'élément de jonction (21) et en ce que les surfaces, qui sont en contact mutuel, sont constituées, à la façon d'une vanne à tiroir, comme des surfaces de commande (27, 50, 55) servant à commuter au moins un canal (25, 48, 26, 49, 18, 52, 53) destiné à la propagation du son.

5. Stéthoscope selon au moins une des revendications 2 à 4, caractérisé en ce que le tube double (11, 12) est relié à l'élément de commutation (37).

6. Stéthoscope selon les revendications 4 et 5, caractérisé en ce qu'il est prévu, sur l'élément de commutation (37), une surface de commande (55) pourvue de deux ouvertures (48, 49), dont chacune est reliée à un tuyau correspondant (11 ou 12) du tube double.
chambres d'écoute (23, 24) de la tête d'écoute (22) à chambre double sont chacune constituées sensiblement comme des évidements ayant une forme sphérique ou en paraboloïde, ces évidements ayant de préférence leurs centres écartés de plus de 10 mm, et se coupant de préférence.

7. Stéthoscope selon la revendication 6, caractérisé en ce que la tête d'écoute (22) à chambre double comporte une surface de commande (27), complémentaire d'une première surface de commande (55) de l'élément de commutation (37), et dans laquelle débouchent deux canaux de liaison (25, 26) menant chacun à l'une des deux chambres d'écoute (23, 24) et en ce que ces canaux de liaison (25, 26) de la tête d'écoute (22) à chambre double peuvent être reliés chacun à une ouverture correspondante (48 ou 49) de l'élément de commutation (37) dans l'une au moins des positions de rotation (fig. 3) de l'élément de commutation (37).

8. Stéthoscope selon la revendication 6 ou 7, caractérisé en ce que l'élément de jonction (21) comporte, de préférence à sa périphérie extérieure, une surface de commande avec deux ouvertures (18), qui sont toutes deux reliées à la chambre d'écoute (16) de la tête d'écoute (13) à une chambre, et en ce que l'élément de commutation (37) comporte une deuxième surface de commande (50), complémentaire de la précédente, avec deux ouvertures de commande (52, 53) reliées chacune à un tuyau (11 ou 12) du tube double, ces ouvertures de commande pouvant être mises en communication avec lesdites ouvertures (18) dans au moins une position de rotation (fig. 5) de l'élément de commutation (37).

9. Stéthoscope selon les revendications 7 et 8, caractérisé en ce que, dans la ou les positions de rotation (fig. 3) dans lesquelles deux chambres d'écoute (23, 24) de la tête d'écoute (22) à chambre double sont reliées chacune, par l'élément de commutation (37), au tuyau correspondant (11 ou 12) du tube double, les ouvertures (18) de la surface de commande prévue sur l'élément de jonction (21) sont bouchées par la deuxième surface de commande (50) de l'élément de commutation (37).

10. Stéthoscope selon les revendications 7 et 8, caractérisé en ce que, dans la ou les positions de rotation (fig. 5) de l'élément de commutation (37), dans lesquelles la chambre d'écoute (16) de la tête d'écoute (13) à une chambre est reliée aux deux tuyaux (11, 12) du tube double, les canaux de liaison (25, 26), menant aux deux chambres d'écoute (23, 24) de la tête d'écoute (22) à chambre double, sont bloqués par la première surface de commande (55) de l'élément de commutation (37).

11. Stéthoscope selon au moins une des revendications 1 à 10, caractérisé en ce qu'un anneau d'étanchéité (29, 34) de préférence en matériau glissant, en particulier en polytétrafluoroéthylène, est disposé entre au moins une tête d'écoute (13, 22) et l'élément de commutation (37), extérieurement aux ouvertures de commande (18, 48, 49).

12. Stéthoscope selon au moins une des revendications 2 à 11, caractérisé en ce qu'il est prévu un dispositif d'arrêt (65 à 68), qui fait arrêter l'élément de commutation (37) dans des positions de rotation déterminées, l'élément de commutation (37) et les têtes d'écoute (13, 22) étant de préférence pourvus de marques (61) à leur périphérie extérieure pour repérer les différentes positions de rotation (fig. 1).

13. Stéthoscope selon au moins une des revendications précédentes, caractérisé en ce que les chambres d'écoute (23, 24) de la tête d'écoute (22) à chambre double sont chacune constituées sensiblement comme des évidements ayant une forme sphérique ou en paraboloîde, ces évidements ayant de préférence leurs centres écartés de plus de 10 mm, et se coupant de préférence.

Fig. 1

Fig. 2

Fig. 3

Fig. 4

Fig. 5

Fig. 6

Fig. 7

Fig. 8

Fig. 9

Fig. 10